Europäisches Patentamt

European Patent Office

Office européen des brevets

(19)

(11) Publication number: **0 264 210**
**A2**

# EUROPEAN PATENT APPLICATION

(21) Application number: **87308745.6**

(22) Date of filing: **02.10.87**

(51) Int. Cl.⁴: **C12M 1/40**

(30) Priority: **15.10.86 US 919032**

(43) Date of publication of application:
**20.04.88 Bulletin 88/16**

(84) Designated Contracting States:
**AT BE CH DE ES FR GB GR IT LI LU NL SE**

(71) Applicant: **Canpolar Inc.**
**Ashley Building 31 Peet Street**
**St John's Newfoundland A1B 3W8(CA)**

(72) Inventor: **Reimer, Ernest M.**
**1 Atlantic Avenue**
**St. John's Newfoundland A1E 1K9(CA)**

(74) Representative: **Matthews, Heather Clare et al**
**Keith W Nash & Co Pearl Assurance House**
**90-92 Regent Street**
**Cambridge CB2 1DP(GB)**

(54) **Chemical probe and enzyme sensor therefor.**

(57) A biochemical probe (10) for determining the freshness of fish or meat, or inspecting the nature of other animal tissues, employs a hypoxanthine enzyme sensor (20) located at a sharp end (16) of the probe. The sensor comprises a reference electrode (24), a pair of sensor electrodes (26), and a membrane (34) that extends over these electrodes to form an outer face of the sensor, this face being a continuation of the surface (18) of the shaft of the probe. The invention is characterized in that the membrane is a thin layer of anodized aluminum oxide which is highly porous and has a large number of small cells (35) extending from the outer face into communication with the electrodes. The cells contain an electrolyte, and those of them in communication with a first one of the sensor electrodes also contain immobilized hypoxanthine oxidase enzymes (37). A comparison of the current flowing between the first sensor electrode and the reference electrode, with the current flowing between the second sensor electrode and the reference electrode, provides an indication of the concentration of hypoxanthine at the outer face of the sensor. If the probe has been inserted into the meat of a fish, a high hypoxanthine concentration would indicate a lack of freshness. The miniturized nature of the sensor, its mounting on the end of a sharpened probe, and the use of a thin layer of highly porous anodized aluminum oxide as the enzyme matrix, achieve a faster response time than hitherto available. The inventive use of anodized aluminum oxide can be applied to a wide range of other chemical (including biochemical) enzyme sensors for mounting in probes or other use.

FIG. 2

# Chemical probe and enzyme sensor therefor

## Field of the Invention

This invention relates to a chemical (including biochemical) probe and to an assembly for use in such a probe as an enzyme (e.g. hypoxanthine or inosine enzyme) mediated sensor utilizing an electrochemical detection technique.

An important use for such a probe is to determine the freshness of fish or animal meat, although use in the examination of other animal or human tissues is also contemplated. Moreover, the probe can be used for detecting slight amounts of various other components such as glucose, urea, uric acid, triglycerides, phospholipids, creatinine, amino acids, lactic acid, xanthine, chondroitin transaminase etc. that may be contained in body tissues or fluids, and in foodstuffs and the like.

## Prior Art

Electrochemical and ion specific sensors have been in use in laboratories for more than 50 years. The commonest sensors are the polarograph and the pH meter both of which rely on electrochemical reactions to sense the concentration of specific ions in aqueous solution. Sensors of this type have been limited primarily to inorganic ions. The recent availability of substrate-specific enzymes has made it possible to devise substrate-specific and sensitive sensors that are based on the detection of reaction byproducts via a conventional pH electrode or polarographic analysis techniques. The simplest embodiment of this technology consists of a pH (or other) electrode with enzymes smeared on the active surface covered by a membrane that retains the enzyme but allows the diffusion of substrate and reaction products. The sensors have typical dimensions of a few centimeters and have response equilibration times of minutes to hours.

More advanced technologies have produced miniature sensors employing field effect transistors that can be introduced into a hollow catheter hypodermic syringe. The sensors and the incorporated membrane are small (dimensions of a few 100 μm) but they are fragile and must be mechanically protected from direct contact with the tissues into which they are inserted. The requisite mechanical protection introduces a "dead space" above the sensor that results in a diffusion barrier and a slow sensor response time. These sensors are relatively expensive as a result of the labour involved in mounting and wiring the microchip in the catheter wall.

It is known to test the freshness of fish by means of a hypoxanthine or inosine enzyme sensor. Typically such a sensor is based on the use of a hypoxanthine oxidase (Hx oxidase) enzyme immobilized on a conventional voltametric oxygen electrode. When a fish dies, decomposition of ATP (adenosine-5'-triphosphate) in the fish meat commences and ADP (adenosine-5'-diphosphate) and AMP (adenosine-5'-monophosphate) are generated. These compounds in turn break down into IMP (inosine-5'-monophosphate), inosine, hypoxanthine, xanthine, and ultimately uric acid. Inosine or hypoxanthine (depending on the species of fish) accumulates with increased storage time, and the concentration of one or other of these compounds provides an indication of freshness. Further details of this action are set out in the literature and, in particular, in a paper entitled "Determination of Hypoxanthine in Fish Meat with an Enzyme Sensor" by E. Watanabe et al., published in the Journal of Food Science Vol 48(2): 496-500 (1983) by the Institute of Food Technologists, page 496.

Since the principle of employing an enzyme electrode for this purpose was proposed a few years ago, a number of devices have been developed and have proved useful, although they have experienced significant practical limitations. The system described in the above-mentioned Watanabe paper employs a so-called Clark type electrode which consists of a platinum cathode, a lead anode, an alkaline (KOH) electrolyte and an oxygen-permeable Teflon™ membrane. An enzyme immobilizing membrane is tightly fixed on the Teflon membrane and is covered with a dialysis outer membrane of cellulose acetate. One of the stated objects of this system is to increase the speed of response and generally to simplify the structure relative to the systems that had been developed earlier. The response time is claimed in the Watanabe paper to be reduced to about 30 seconds. This compares favourably with the 10 minutes that had previously been required, but it is still only suited to laboratory conditions and limits the number of samples than can be tested per hour.

A range of other electrochemical sensors is described in a paper entitled "Electrochemical Sensors in Clinical Chemistry: Yesterday, Today, Tomorrow" by John D. Czaban published in Analytical Chemistry, Vol. 57, No. 2, Feb. 1985 p. 345.

Sensors required for many industrial applications, such as food processing, must meet cost and performance standards that cannot be delivered by existing technologies. For fish quality monitoring the response equilibration time must be less than one second and the cost must be less than one cent. This cost specification implies a sensor than can be sold for $10.00 and that can be used for at least 1,000 assays. The response time requirement implies a sensor that is insertable into tissue and one that has no mechanical protection that will separate it from the sample by more than a few tens of μm. Normal membrane materials e.g. cellulose, Teflon, etc., are not durable enough at 10 μm thickness to tolerate repeated insertion into tissues.

## Summary of the Invention

The object of the present invention is to overcome the above-described difficulties and provide a chemical enzyme sensor that has a fast response time, e.g. no more than about one second, is relatively cheap to manufacture, and is rugged enough to withstand repeated use while retaining its reliability of operation.

To this end, the invention provides a chemical enzyme sensor that employs an outer membrane comprising a thin layer of anodized aluminum oxide. This material has a large number of small cells that are ideal for containing an electrolyte and for the immobilization of enzymes therein.

The electrochemical anodic oxidation of aluminum creates a porous structure in which the pore (cell) size and depth can be exactly controlled. The structure created in this way has geometric features many orders of magnitude smaller than could be obtained by masking and etching techniques. As a result, this structure is ideal for enzyme immobilization. The creation of individual electrochemical cells provides an ideal enzyme sensor configuration of a size much smaller than could be achieved by other technologies, and at a low cost.

In addition, anodized aluminum oxide has a very high abrasion resistance, being used as a coating in high wear machinery such as hydraulic pistons.

Thus, the technical problems mentioned above were size (response time limitation) and cost (fabrication and multiple use limitation). Both of these problems can be effectively solved through the use of a controlled pore size, anodized aluminum oxide, membrane-immobilization matrix in place of the polymeric materials normally used for the sensor. Another advantage of the use of anodised aluminum oxide is that it is very low cost material that can be readily fabricated using conventional techniques. See the textbook "Anodic Oxidation of Aluminum and its Alloys" by V.F. Henley, published by Pergamon Press, and especially Chapter 14 entitled "The Properties of Anodized Aluminum."

Miniaturization of the sensor permits the probe to take the form of a hand held device that can be readily inserted into an object under examination, e.g. a fish, in much the same manner as a hypodermic needle, while providing the user with a virtually instantaneous indication of the status of the object, e.g., in the case of a fish, the degree of freshness of its meat.

A specific form of the invention described in detail below provides a hand-held chemical probe having a shaft terminating in a sharp end for penetrating tissue to be examined, and an enzyme sensor recessed in a very shallow cavity in a surface of the shaft adjacent the sharp end. This sensor comprises a reference electrode, a pair of sensor electrodes, and a membrane extending over these electrodes to form an outer face of the sensor, such face preferably forming a continuation of the shaft surface. The membrane comprises a very thin layer of anodized aluminum oxide having a large number of small cells extending from the outer face into communication with respective ones of the electrodes. These cells contain an electrolyte, and those in communication with a first one of the sensor electrodes also contain immobilized enzymes. The electrodes can be electrically connected to a circuit for applying a voltage between the reference electrode and each of the sensor electrodes and for comparing the currents flowing in the respective sensor electrodes.

The invention not only relates to such probe but also to an enzyme sensor per se for use in such a probe.

Furthermore, the invention also relates to an assembly for use in preparing such an enzyme sensor.

## Brief Description of the Drawings

Figure 1 is a small scale, perspective view of a biochemical probe according to a preferred embodiment of the invention;

Figure 2 is a larger scale, plan view of this probe;

Figure 3 is a diagrammatic cross-section, on a very much enlarged scale and with the vertical distances exaggerated, of a sensor embodied in the probe;

Figure 4 is a still further enlarged cross-section of a fragment of this sensor, this time with the vertical scale reduced;

Figure 5 is a fragment of a modification of Figure 4; and

Figure 6 is a diagrammatic electric circuit.

## Detailed Description of the Preferred Embodiment

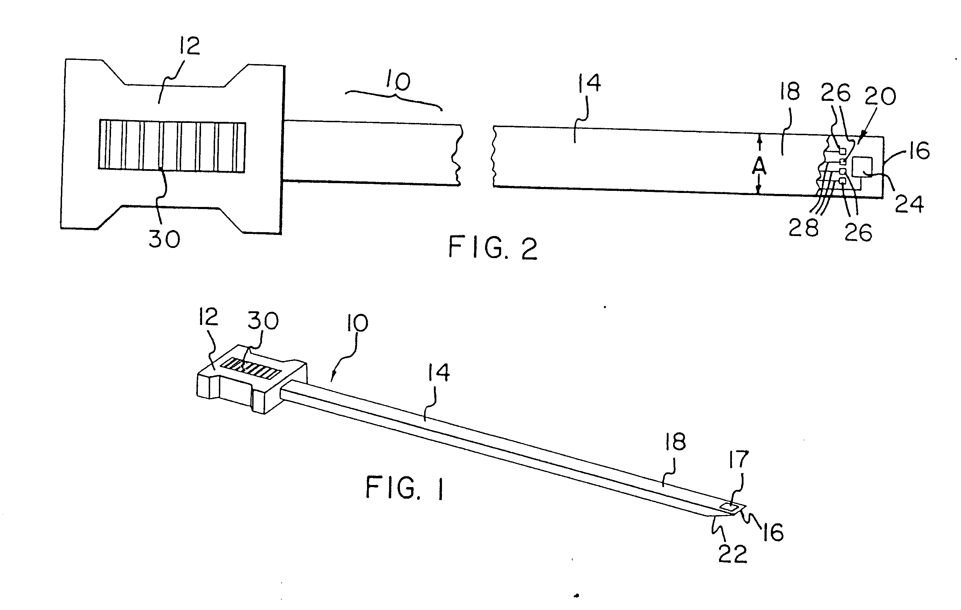

Figures 1 and 2 show a probe 10 with an elastomeric connector assembly 12 and a shaft 14, the latter terminating in a sharp end 16 (typically 1 mm in the width dimension A), and having a sensor 20 located in a shallow cavity 17 in its upper surface 18 just inwardly from the sharp end 16. Alternatively, the sensor 20 can be set in the slanting surface 22 that together with the upper surface 18 defines the sharp end 16. In either case, the outer face of the sensor will be brought into intimate contact with the tissue under inspection, once the tip of the probe has penetrated the surface of such tissue.

The sensor 20 shown in Figure 2 has a silver reference electrode 24 and four platinum sensor electrodes 26 connected to respective copper conductors 28 that extend to contacts 30 in the connector assembly 12, these contacts providing means for mating the probe to a hand held electronic control (not shown).

Each platinum electrode 26 is very small, typically having a dimension in each direction of the order of 10 $\mu$m, i.e. an area of 100 $\mu$m$^2$. The silver electrode 24 will be large in comparison, in order to avoid electrode polarization effects. A first one of the platinum electrodes 26 communicates with a body of electrolyte containing appropriate enzymes, to become a so-called $PtO_2$-E sensor, while a second of the electrodes 26 has an identical structure but without the enzymes, and serves the function of a so-called $PtO_2$ sensor. The latter sensor provides a reading of local oxygen concentration, which is needed for the interpretation of the results. The other two electrodes 26 shown in Figure 2 are spares, and can be used with a different enzyme to achieve a different measurement. Alternatively, the third and fourth electrodes 26 can be dispensed with.

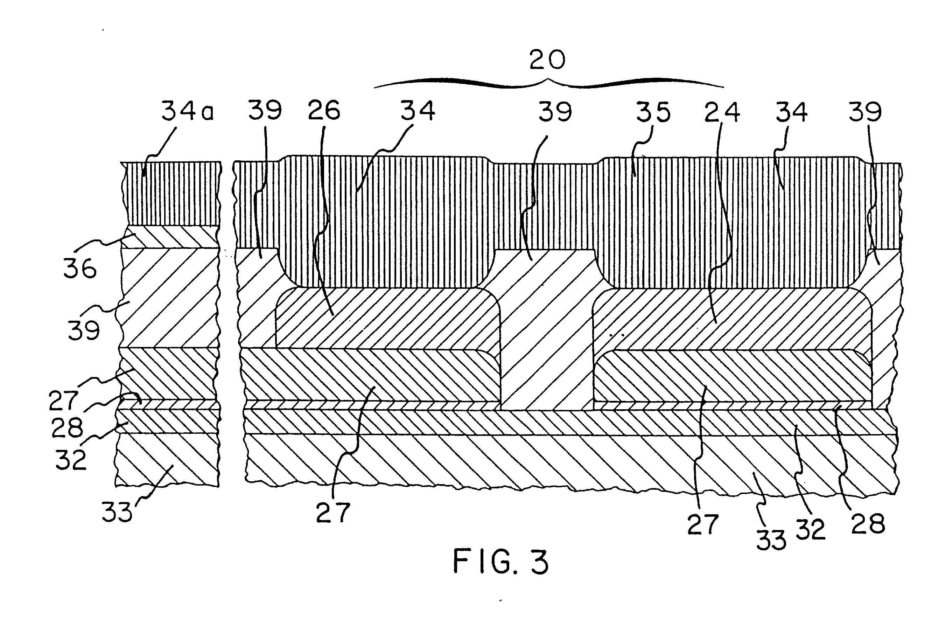

Figure 3 shows a section through the sensor 20 and specifically one platinum electrode 26 and the silver electrode 24 connected to respective copper conductors 28 through intermediate gold members 27. Numeral 32 shows an insulating layer and 13 represents the stainless steel of the shaft 14 of the probe, which surrounds the sensor 20 in the shallow cavity 17.

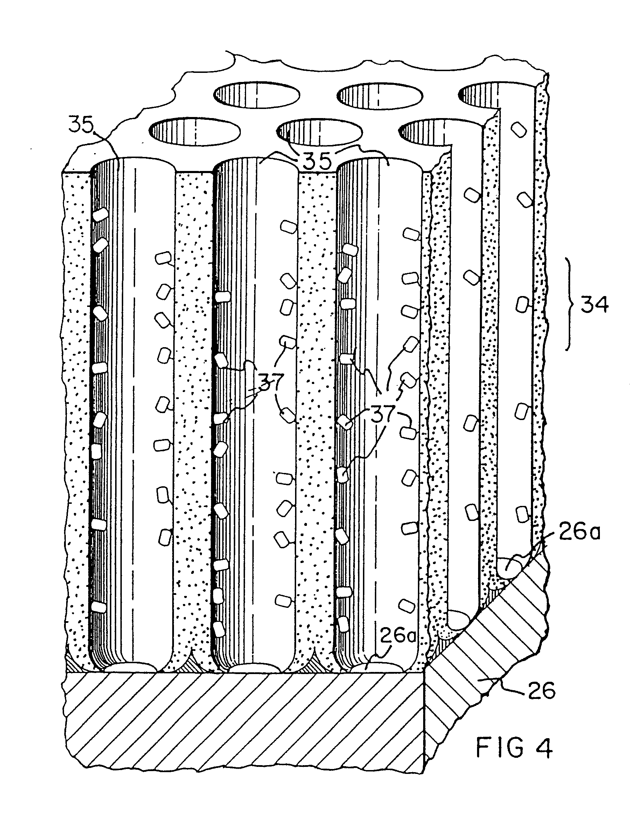

Superimposed on the active electrodes 24 and 26, and covering the entire sensor 20, there is an outer layer or membrane 34 of anodized aluminum oxide. This layer 34 is highly porous (Figure 4), containing a very large number of very small cells 35. Typical dimensions for a cell 35 would be a length of about 1 $\mu$m and a diameter of 0.1 to 0.05 $\mu$m. The latter dimensions are ideal for occlusion of unwanted cellular debris while permitting effective travel of hypoxanthine and oxygen and effectively immobilizing the enzymes. The platinum of the electrode 26 is shown exposed at 26a at the inner end of each cell 35. In a similar manner, the inner end of each cell 35 over the silver electrode 24 will communicate directly with a layer of silver chloride (not shown) overlying the electrode 24. This layer can be either deposited in the cells or created electrochemically.

An alternative construction may be employed to avoid problems relating to the adhesion between different layers of metal and oxide during the fabrication of the sensor, namely:

The basic circuit conductors and electrode pads may be fabricated from a thick aluminum layer which is then anodized to the desired length. A catalytic electrode surface of noble metal is then deposited onto/into the anodized aluminum layer. This fabrication method would provide a maximum bonding strength between the anodized oxide structure and the underlying circuit conductor.

During fabrication of the sensor 20, which can be carried out using conventional deposition and etching techniques of the type employed in the manufacture of integrated circuits and the like, the anodization step will be carried out on an upper aluminum layer so as to be carried completely through the metallic aluminum in those areas overlying the platinum and silver electrodes 24, 26, i.e. to form the membrane 34, but to stop short in other areas of the probe. See the shallower layer 34a of anodized aluminum oxide, beneath which there remains a layer of aluminum 36 that in turn overlies an insulating layer 39 of unanodized aluminum oxide, silicon oxide or tantalum oxide applied by one of the known deposition techniques.

The outer face of the anodized layer serves as an abrasion resistant surface. The membrane 34 also provides an ideal immobilization matrix for the sensor enzymes 37 (Figure 4). The enzymes 37 will be co-valently immobilized inside the individual cells 35 of the membrane 34 in a physiological saline solution or other appropriate electrolyte, such as a polymeric gel.

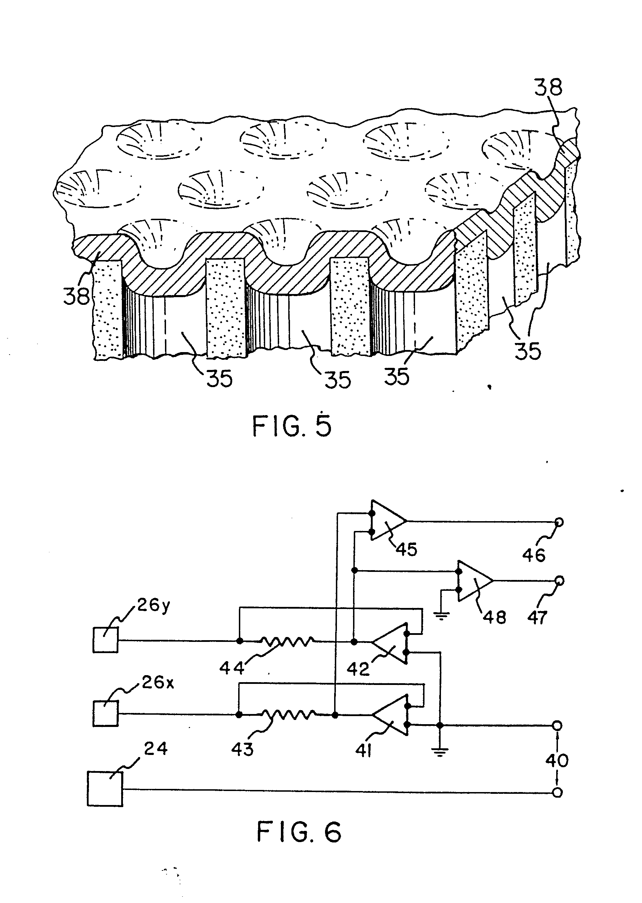

The cells can be left open (Figure 4) or, as an optional additional feature, can be covered by a very thin secondary membrane 38 (Figure 5), such as a lipid bilayer with a suitable ion and molecular permeability, or a thin Teflon layer, or a layer of solvent deposited polymers or of a vacuum deposited metal or oxide. A typical thickness for such a secondary membrane 38 would be 0.02 $\mu$m which is so thin that the upper ends of the cells 35 can still be considered as effectively extending inwardly from the outer face of the sensor 20. The primary function of such a secondary membrane 38 would be to enhance the stability and lifetime of the sensor by additional exclusion of unwanted contamination by biological materials. Although this secondary membrane 38 will not have the durability of the anodized aluminum oxide membrane 34, it will, nevertheless, have a high mechanical integrity by virtue of the very small size of the cell openings that it bridges.

In operation, the sensor functions chemically and electrically in essentially the same basic manner as in the prior art - as described in the Watanabe paper referred to above. Further details of these functions are given in another paper by E. Watanabe et al entitled "Enzyme sensor for hypoxanthine and inosine determination in edible fish", published in Applied Microbiology and Biotechnology (1984) 19:18-22 by Springer-Verlag, page 97.

Figure 6 shows a simplified electrical diagram, A D.C. voltage applied at 40 from a battery (not shown) is amplified by a first amplifier 41 and then applied between the $PtO_2$-E sensor electrode 26x and the reference electrode 24, and by a second amplifier 42 and applied between the $PtO_2$ sensor electrode 26y and the reference electrode 24. The respective currents as detected by the voltages across resistors 43 and 44 are compared in a comparator 45 to give an output at terminal 46 indicative of the hypoxanthine concentration. The oxygen concentration appears at terminal 47 through amplifier 48 from resistor 44.

The respect in which the present invention is an improvement relative to the prior proposals resides basically in its much improved speed of operation (30 or more times faster), which result flows in part from the minaturization with the very short diffusion distances thus involved. The improved speed of operation is also a consequence of the virtually direct contact between the tissue under inspection which lies against the outer face of the membrane 34 and the electrodes 24, 26. The highly porous nature of the anodized aluminum oxide layer (membrane) 34 ensures this virtually direct contact, and this is still essentially true even when the further outer membrane 38 (Figure 5) is employed, in view of the extreme thinness of this membrane 38. The cells 35 in the membrane 34 are also of appropriate size for providing an excellent matrix for immobilizing the enzymes.

At the same time, the anodized aluminum oxide layer will provide a highly abrasion resistant outer face. This feature is important for achieving a probe that will continue to function reliably after it has been used a large number of times to penetrate tissue samples under inspection.

In order to reduce electrical noise interference (since the currents in the sensor will be very small, e.g. of the order of $10^{-12}$ amperes), the electrodes 26 will be similar to each other in size and arrangement and will be symmetrically disposed in relation to the electrode 24. Moreover, these electrodes and the conductors extending to them will have shielding (not shown). The shielding will serve the further function of reducing leakage currents from the high impedance circuit (Figure 6) which is housed in the handle 12.

It is to be understood that the present invention is not limited to the particular type of sensor illustrated in the drawings. There have been many recent developments in chemically sensitive solid state devices, particularly ion selective field effect transistors (ISFETS). Also known are potentiometric sensors, pyroelectric enthalpimetric sensors and surface acoustic wave sensors. Surface acoustic wave (SAW) sensors detect modifications to surface wave propagation characteristics. Biosensors or chemosensors based on SAW devices use a selective affinity layer to detect specific compounds. Many of the enthalpimetric sensors which have been fabricated rely on substrate specific enzymes which catalyse an exothermic reaction. The sensor itself responds to minute temperature differences. Many different types of potentiometric sensors have been designed, some commercially available. The detection specifically of potentiometric biosensors is often mediated by a substrate specific oxidase or peroxidase enzyme.

The ability of these devices to sense specific biochemical compounds or metabolites in complex media makes them ideal for biomedical monitoring and diagnostics. It also makes them quite attractive for food process control applications. A state of the art review of bioelectrochemical sensors specific for substrates such as sucrose, monoamine, phopholipid, cholesterol, etc. is contained in a paper by S. Suzuki and I. Karube published in Applied Biochemistry and Bioengineering Vol. 3, AP, NY (1981).

The present invention of anodized aluminum oxide is believed applicable to any of these chemical sensors in which there is need for an abrasion resistant, thin, outer membrane of porous material that is well adapted to enzyme immobilization.

## Claims

1. A chemical sensor (20) having an outer membrane (34), characterised in that the membrane comprises a thin layer of anodized aluminum oxide having a large number of small cells (35) for containing an electrolyte and for immobilizing enzymes (37).

2. An assembly for use as an enzyme sensor (20) in a chemical probe (10), said assembly comprising a reference electrode (24), at least one sensor electrode (26), and a membrane (34) extending over said electrodes, characterised in that the membrane comprises a thin layer of anodized aluminum oxide having a large number of small cells (35) extending from an outer surface into communication with respective ones of said electrodes.

3. An assembly according to claim 2 having a pair of said sensor electrodes.

4. An enzyme sensor (20) for use in a chemical probe (10), comprising a reference electrode (24), a pair of sensor electrodes (26), and a membrane (34) extending over said electrodes to provide an outer face for the sensor, characterised in that the membrane comprises a thin layer of anodized aluminum oxide having a large number of small cells (35) extending from said outer face into communication with respective ones of said electrodes, said cells containing an electrolyte and those cells in communication with a first one of said sensor electrodes also containing immobilized enzymes (37).

5. In a chemical probe (10) having a shaft (14) terminating in a sharp end (16) for penetrating tissue to be examined,

(a) an enzyme sensor (20) on a surface (18) of the shaft adjacent said end;

(b) said sensor comprising a reference electrode (24), a pair of sensor electrodes (26), and a membrane (34) extending over said electrodes to form an outer face of the sensor;
characterised in that

(c) said membrane comprises a thin layer of anodized aluminum oxide having a large number of small cells (35) extending from said outer face into communication with respective ones of said electrodes;

(d) said cells contain an electrolyte, and those cells in communication with a first one of said sensor electrodes also contain immobilized enzymes (37); and

(e) said electrodes are connected to a circuit (40-45) for applying a voltage between the reference electrode and each of the sensor electrodes and for comparing the currents flowing in the respective sensor electrodes.

6. A probe according to claim 5, characterized in that the outer face of the sensor forms a continuation of said shaft surface.

7. A probe according to claim 5, characterised in that the anodized aluminum oxide membrane has a thickness no more than 10 $\mu$m.

8. A probe according to claim 7, characterised in that said thickness is approximately 1 $\mu$m.

9. A probe according to claim 5, characterised in that the sharp end of the probe has a transverse dimension of about 1 mm, the sensor electrodes have an area of about 100 $\mu$m$^2$, the anodized aluminum oxide membrane has a thickness of about 1 $\mu$m, and the cells have a diameter of about 0.1 to 0.05 $\mu$m.

FIG. 2

FIG. 1

FIG. 3

FIG 4

FIG. 5

FIG. 6